# EUROPEAN PATENT SPECIFICATION

(11) **EP 1 463 883 B1**
(45) Date of publication and mention of the grant of the patent: **11.07.2012**
(21) Application number: 02790006.7
(22) Date of filing: 04.12.2002
(51) Int. Cl.: F02M 23/04, F02M 15/00, F02M 17/28, F02M 11/00, B01D 47/00, C10J 1/10, F24F 3/14, A61M 16/00, A61M 15/00, B05D 7/14

(54) **AEROSOL GENERATOR HAVING A MULTIPLE PATH HEATER ARRANGEMENT AND METHOD OF USE THEREOF**
AEROSOLERZEUGER MIT EINER MEHRPFADIGEN HEIZANORDNUNG UND VERWENDUNGSVERFAHREN DAFÜR
GENERATEUR D'AEROSOL COMPORTANT UN DISPOSITIF CHAUFFANT A TRAJETS MULTIPLES ET PROCEDE D'UTILISATION ASSOCIE

(30) Priority: 06.12.2001 US 3438
(43) Date of publication of application: 06.10.2004
(73) Proprietor: Philip Morris USA Inc., Richmond, VA 23230 (US)
(72) Inventor: SPRINKEL, JR., F., Murphy, Glen Allen, VA 23059 (US); NICHOLS, Walter, A., Chesterfield, VA 23832 (US)
(74) Representative: Marlow, Nicholas Simon
(86) International application number: PCT/US2002/038681
(87) International publication number: WO 2003/050405

(56) References cited:
- WO-A-01/81182
- US-A- 4 682 010
- US-A- 5 743 251
- US-A- 5 795 626
- US-A- 6 155 268
- US-B1- 6 234 167
- US-B1- 6 234 167

## Description

### BACKGROUND OF THE INVENTION

### 1. Field of the Invention

The present invention relates generally to aerosol generators and, more particularly, to aerosol generators which include a heater for volatilizing liquid material. The present invention also relates to methods for generating an aerosol. The present invention has particular applicability to the generation of aerosols containing medicated material.

### 2. Description of the Related Art

Aerosols are gaseous suspensions of fine solid or liquid particles and are useful in a wide variety of applications. For example, medicated liquids and powders may be administered in aerosol form. Such medicated aerosols include, for example, materials which are useful in the treatment of respiratory ailments, in which case the aerosols may be inhaled into a patient's lungs. Aerosols may also be used in non-medicinal applications including, for example, dispensing air fresheners and insecticides and delivering paints and/or lubricants.

In aerosol inhalation applications, it is typically desirable to provide an aerosol having an average mass median particle diameter of less than 2 microns to facilitate deep lung penetration. Most known aerosol generators are incapable of generating aerosols having an average mass median particle diameter less than 2 microns. Also, in certain applications, it is generally desirable to deliver medicated material at high flow rates, for example, above 1 mg per second. Most known aerosol generators suited for delivering medicated material are incapable of delivering material at such high flow rates while maintaining a suitable average mass median particle diameter. In addition, most known aerosol generators deliver an imprecise amount of aerosol compared with the amount of aerosol that is intended to be delivered.

Commonly owned U.S. Patent Nos. 5,743,251 and 6,234,167, disclose aerosol generators designed for volatilizing a liquid and ejecting the volatilized liquid into the atmosphere. The volatilized liquid subsequently condenses, thereby forming an aerosol. Such aerosol generators may utilize resistance heating materials to volatilize the liquid. However, generators having a single zone wherein the liquid is heated may not provide optimal delivery of the volatilized liquid.

In light of the foregoing, there exists a need in the art for the provision of an aerosol generator which provides improved aerosol delivery of volatilized liquid.

### SUMMARY OF THE INVENTION

According to a first aspect of the present invention as claimed, an aerosol generator includes a liquid supply, a flow passage including at least first and second flow paths in fluid connection at upstream ends thereof, a single inlet in fluid communication with the liquid supply for supplying the first and second flow passages and at least one outlet, and a heater arrangement including first and second heating sections, the first heating section being adapted to heat liquid in the first flow path sufficiently to vaporize the liquid so as to form a vaporized liquid ejected from the at least one outlet, and the second heating section being adapted to heat liquid in the second flow path sufficiently to vaporize liquid so as to form a vaporized liquid ejected from the at least one outlet.

The invention also provides a method for generating an aerosol comprising supplying material in a liquid form to an aerosol generator comprising a flow passage having an inlet in fluid communication with a liquid supply, the flow passage including at least first and second flow paths in fluid connection at upstream ends thereof and at least one outlet; and heating the liquid in the first and second flow paths to a temperature sufficient to volatilize the liquid and eject volatilized liquid from at least one outlet. The inlet of the flow passage is a single inlet for supplying liquid to the first and second flow passages.

Preferably, the heating step is conducted with a multi-path heater arranged to volatilize fluid, wherein the heater includes at least first and second heating sections, the first heating section being adapted to heat liquid in the first flow path sufficiently to vaporize the liquid so as to form a vaporized liquid ejected from the at least one outlet, and a second heating section being adapted to heat liquid in the second flow path sufficiently to vaporize the liquid so as to form a vaporized liquid ejected from the at least one outlet. The heater arrangement of the aerosol generator may be activated to provide a differential heating rate in the first and second flow passages, and to direct a smaller amount of volatilized fluid out of the first flow path, prior to directing the bulk of volatilized fluid out of the second flow path.

### BRIEF DESCRIPTION OF THE DRAWINGS

The objects and advantages of the invention will become apparent from the following detailed description of the preferred embodiments thereof in connection with the accompanying drawings, in which:
FIGS. 1 is a schematic view of an aerosol generator of an inhaler according to a first embodiment of the present invention;
FIG. 2 is a top plan view of a base plate of a multiple path heater arrangement according to the present invention;
FIG. 3 is a side sectional view along line A-A of a base plate according to the present invention; and
FIG. 4 is a top plan view of an assembled multiple path heater arrangement according to an embodiment of the present invention.

### DETAILED DESCRIPTION OF THE PREFERRED EMBODIMENTS OF THE INVENTION

The present invention provides improvements in delivery of volatilized liquid from an aerosol generator via a multi-path heating arrangement which can deliver low volume mass per inhalation cycle, volatilize low solute containing solutions, minimize overheating, minimize power requirements, form volatilized liquid more quickly and/or form a predetermined amount of aerosol in a shorter time than in aerosol generators utilizing a single flow passage/heater arrangement. The invention is described with reference to embodiments shown in the drawing figures, wherein like reference numerals designate identical or corresponding elements throughout the several figures.

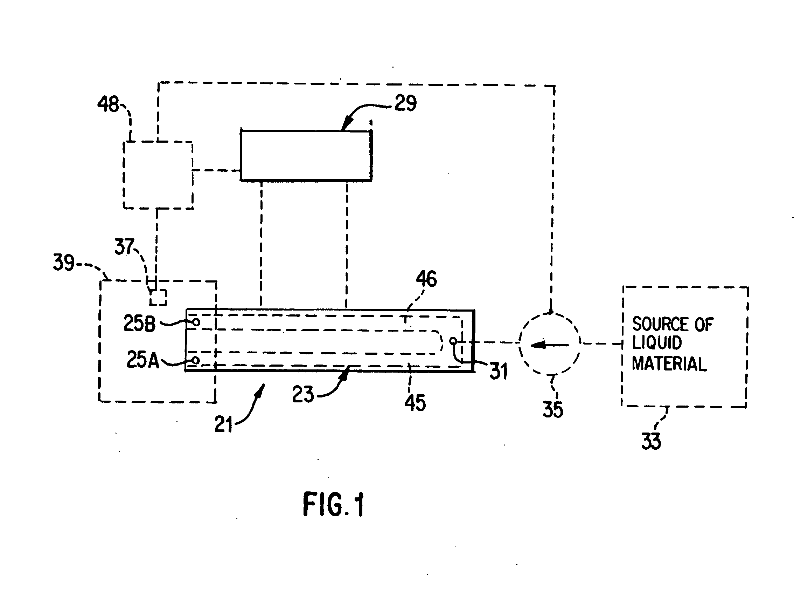

An aerosol generator 21 of an inhaler according to a first embodiment of the present invention is shown with reference to Figure 1. The aerosol generator 21 includes a liquid supply 33 which is in direct communication with a multiple path time than in aerosol generators utilizing a single flow passage/heater arrangement. The invention is described with reference to embodiments shown in the drawing figures, wherein like reference numerals designate identical or corresponding elements throughout the several figures.

An aerosol generator 21 of an inhaler according to a first embodiment of the present invention is shown with reference to Figure 1. The aerosol generator 21 includes a liquid supply 33 which is in direct communication with a multiple path heater arrangement 23. The heater arrangement 23 is connected to a power supply 29, preferably a DC power source such as a battery. Liquid from liquid source 33 is delivered to flow paths 45 and 46, by any suitable arrangement such as a syringe pump, pressurized container, valve arrangement or the like. In the embodiment shown, a valve 35 is used to deliver a predetermined amount of liquid to inlet 31 of a flow passage which branches into flow paths 45,46. Activation of the valve can be controlled by a controller 48 upon receiving a signal from an optional puff activated sensor 37. The controller also activates heater 23 by supplying power from power supply 29 whereby vaporized liquid is ejected from outlets 25A,25B and/or aerosol is formed in optional mouth piece 39 for inhalation by a user of the device. If desired, a single outlet may be used in lieu of the two outlet arrangement.

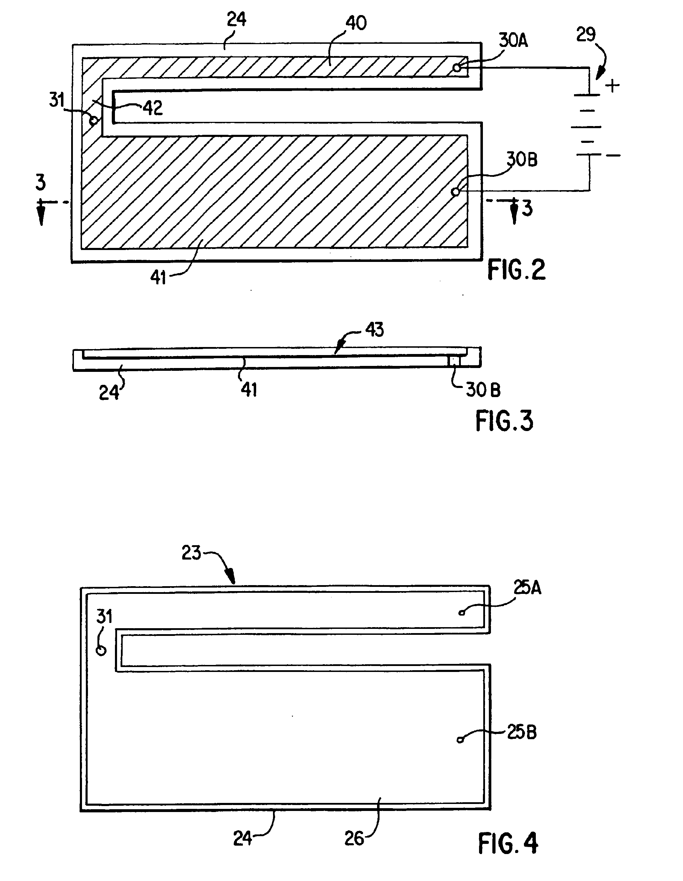

Figures 2-4 show a multi-path heater arrangement according to a preferred embodiment of the invention wherein Figure 2 shows a top view of a base plate 24, Figure 3 shows a side view of the base plate 24, and Figure 4 shows a top view of a top plate 26 assembled to the base plate. The base plate 24 and top plate 26 when assembled form the multilayered composite heater arrangement 23 shown in Figure 1.

The aerosol generator 21 can produce an aerosol from a fluid in liquid form by volatilizing the fluid at a differential heating rate within the flow paths 45 and 46. The flow paths 45,46 can have any desired configuration. For example, flow path 45 can comprise a straight and uniform cross-sectioned channel which is parallel to flow path 46 as shown in Figure 1. However, the flow paths could have non-uniform cross-sections, could be non-parallel and/or could be non-linear flow paths.

Figure 2 shows an arrangement wherein the multiple path heater comprises at least two heating zones 40,41. The first heating zone 40 is located along the first flow path 45 which preferably has a smaller cross-sectional area than the second flow path 46. The smaller cross-sectional area of the first flow path 45 allows for faster heating of the fluid which passes through the multiple path heater 23. As such, when the volatilized fluid enters through the liquid inlet hole 31 and flows through the first and second flow paths 45 and 46, a preliminary amount of volatilized fluid will be produced within the smaller cross-sectional area defined by the first flow path 45 and delivered to outlet 25A, prior to volatilization of fluid in the second flow path 46. Thus, the aerosol generator can deliver an aerosol to a user of the device within a short time of actuation of the heater. While the preliminary amount of volatilized fluid is formed, the second heating section can heat sufficiently to deliver a bulk amount of volatilized fluid to outlet 25B.

The flow paths 45,46 can be formed in a ceramic or polymer base plate 24 by molding, machining or other suitable technique. For example, the base plate 24 can be a green ceramic tape of alumina and the flow paths can be press formed into the ceramic tape. Alternatively, the base plate 24 can be a sintered ceramic plate and the flow paths 45,46 can be laser machined into the plate. The flow paths can have any desired configuration and/or dimensions in terms of length, width and depth. For example, the flow paths 45,46 can be parallel to each other with capillary dimensions, e.g., a depth of 0.01 to 10 mm, preferably 0.05 to 1 mm, and more preferably about 0.1 to 0.5 mm with the width and length of the flow path being any suitable dimensions, e.g., width of 1 mm or more and length of 10 mm or more. The width of flow path 46 is preferably 2 to 10 times greater than that of flow path 45, e.g., flow path 46 can be around 4 times wider than flow path 45. Alternatively, the smaller capillary passage can be defined by transverse cross sectional area of the passage which can be 8 x 10⁻⁵ to 80 mm², preferably 2 x 10⁻³ to 8 x 10⁻¹ mm² and more preferably 8 x 10⁻³ to 2 x 10⁻¹ mm².

The power supply is preferably a battery operated by a controller and connected to the multiple path heater 23 via electrical feedthroughs 30A and 30B. This will allow for a continuous electrical circuit within the multiple path heater and faster heating of the heating zone 40 due to its smaller cross section. The heating zones 40,41 and optional intermediate section 42 can comprise a coating of resistance heating material located in flow paths 45,46. For example, a resistance heating material such as platinum can be deposited such as by sputtering on surfaces of the base plate 24 defining the flow paths 45,46. However, the heater can comprise a layer or layers of heating material on outer surfaces of top and/or bottom plates 24,26. Liquid from a fluid supply can be supplied continuously or intermittently to inlet hole 31. For inhaler devices, the inlet hole can have a size of 0.05 to 5 mm, preferably 0.1 to 1 mm. As the liquid enters into the fluid inlet hole 31, the continuous electrical circuit allows for the heating of the heating zones 41,42 within fluid channel 43 as indicated in Figure 3. Heating zones 40,41 can be interconnected by intermediate zone 42 in the case where the heating zones 40,41 are formed from a continuous layer of resistance heating material. However, the heating zones 40,41 could be formed from discrete sections of heater material in which case separate electrical connections would be attached to each heater section.

Figure 4 shows a top plate 26 assembled on the base plate 24. The top and base plates can be held together with a suitable adhesive such as cement, epoxy, metallized glass, brazing material or the like. The heater arrangement 23, which includes first and second heating sections 40 and 41, is adapted to heat liquids in a first flow path 45 sufficiently to vaporize the liquid so as to form a vaporized liquid ejected from the first outlet 25A. The second heating section 41 is adapted to heat liquid in the second flow path 46 sufficiently to vaporize the liquid so as to form a vaporized liquid ejected from the second outlet 25B. For inhaler devices, the outlets 25A and 25B can be round holes having a diameter of 0.05 to 5 mm, preferably 0.1 to 1 mm. This arrangement allows for rapid heating of the liquid in the first flow path 45.

While the invention has been described in detail with reference to preferred embodiments, it will be apparent to one skilled in the art that various changes can be made, and equivalents employed, without departing from the scope of the invention.

## Claims

1. An aerosol generator (21) for generating vaporized fluid comprising:
a liquid supply (33);
a flow passage including at least first (45) and second (46) flow paths, an inlet (31) in fluid communication with the liquid supply (33) and at least one outlet (25A,B);
a heater arrangement (23) including first (40) and second (41) heating sections, the first heating section (40) being adapted to heat liquid in the first flow path (45) sufficiently to vaporize the liquid so as to form a vaporized liquid ejected from the at least one outlet (25A), and the second heating section (41) being adapted to heat liquid in the second flow path (46) sufficiently to vaporize the liquid so as to form a vaporized liquid ejected from the at least one outlet (25B)
**characterised in that** the first (45) and second (46) flow paths of the flow passage are in fluid connection at upstream ends thereof and **in that** said inlet is a single inlet (31) for supplying liquid to the first and second flow paths

2. An aerosol generator (21) according to claim 1 wherein the heater arrangement (23) comprises at least one layer of resistance heating material and/or the at least first (45) and second (46) flow paths have capillary dimensions.

3. An aerosol generator (21) according to claim 2 wherein the first (40) and second (41) heating sections have the same or different electrical resistance.

4. An aerosol generator (21) according to claim 2 or 3 wherein the resistance heating material comprises a platinum coating.

5. An aerosol generator (21) according to claim 2, 3 or 4 wherein the heater arrangement (23) includes an intermediate section of resistance heating material extending between the first (40) and second (41) heating sections.

6. An aerosol generator (21) according to claim 2, 3, 4 or 5 wherein the first (40) and second (41) heating sections are powered by a single electrical power source (29).

7. An aerosol generator (21) according to any preceding claim comprising first (25A) and second (25B) outlets.

8. An aerosol generator (21) according to any preceding claim wherein the first heating section (40) has a different cross-sectional area than the second heating section (41).

9. An aerosol generator (21) according to claim 8 wherein the first heating section (40) has a smaller cross-sectional area than the second heating section (41) such that the first heating section (40) volatilizes liquid in the first flow path (45) faster than the second heating section (41) volatilizes liquid in the second flow path (46) when liquid is supplied to the first and second flow paths.

10. An aerosol generator (21) according to any preceding claim further comprising a controller (48), valve (35) and a sensor (37), the sensor (37) detecting a delivery condition corresponding to delivery of a predetermined volume of aerosol, the controller (48) being programmed to open the valve (35) so as to deliver liquid to the first (45) and second (46) flow paths when the delivery condition is sensed by the sensor (37) and to activate the heating sections (40,41) to volatilize liquid in the first (45) and second (46) flow paths.

11. An aerosol generator (21) according to any preceding claim wherein the first heating section (40) is thermally isolated from the second heating section (41).

12. An aerosol generator (21) according to any of claims 1 to 6 wherein the first heating section (40) is thermally integrated with the second heating section (41).

13. An aerosol generator (21) according to any preceding claim wherein the first (40) and second (41) heating sections have the same or different thermal masses.

14. An aerosol generator (21) according to any preceding claim further comprising a mouthpiece of an inhaler, the at least one outlet (25A,B) being located inside the mouthpiece so as to form an aerosol within the mouthpiece when volatilized liquid material is ejected from the at least one outlet (25A,B).

15. An aerosol generator (21) according to any preceding claim wherein the first flow path (45) is sized to hold less than one-half an amount of liquid contained in the second flow path (46).

16. An aerosol generator (21) according to any preceding claim wherein the first (40) and second (41) heating sections eject different volumes of vaporized liquid from the at least one outlet (25A,B) during delivery of a fixed volume of liquid to the flow passage.

17. An aerosol generator (21) according to any preceding claim wherein the first (45) and second (46) flow paths comprise first and second channels between a base plate (24) and a cover plate (26).

18. An aerosol generator (21) according to claim 17 wherein the first heating section (40) comprises a layer of resistance heating material in the first channel and the second heating section (41) comprises a layer of resistance heating material in the second channel.

19. An aerosol generator (21) according to any preceding claim wherein the first heating section (40) is powered independently from the second heating section (41).

20. An aerosol generator (21) according to any preceding claim wherein the first (40) and second (41) heating sections comprise planar strips of electrically resistive heating material.

21. An aerosol generator (21) according to claim 20 wherein the strips of electrically resistive heating material are parallel to each other.

22. An aerosol generator (21) according to claim 20 or 21 wherein the first (40) and second (41) heating sections are interconnected by an intermediate section (42) comprising a planar strip of electrically resistive heating material.

23. An aerosol generator (21) according to any preceding claim wherein the first (45) and second (46) flow paths comprise first and second channels in a ceramic substrate.

24. An aerosol generator (21) according to claim 23 wherein the said channels in the ceramic substrate are less than or equal to 10 mm in depth.

25. An aerosol generator (21) according to claim 23 or 24 wherein the first (40) and second (41) heating sections comprise resistance heating material in the channels.

26. An aerosol generator (21) according to claim 23, 24 or 25 wherein at least one electrical feedthrough (30A,B) extends through the ceramic substrate and supplies power to the heating sections.

27. An aerosol generator (21) according to claim 23, 24, 25 or 26 comprising a first feedthrough (30A) connected to a downstream end of the first heating section (40) and a second feedthrough (30B) connected to a downstream end of the second heating section (41).

28. A method for generating an aerosol, comprising:
(a) supplying a material in liquid form to an inlet (31) of an aerosol generator (21) having a flow passage which includes first (45) and second (46) flow; and
(b) heating the liquid in the first (45) and second (46) flow paths to a temperature sufficient to volatilize the liquid and eject volatilized liquid from at least one outlet (25A,B)
**characterised in that** the first (45) and second (46) flow paths of the flow passage are in fluid connection at upstream ends thereof and **in that** said inlet is a single inlet (31) for supplying liquid to the first and second flow paths.

29. A method according to claim 28 wherein the heating is carried out by energizing first (40) and second (41) heating sections, the first flow path (45) being smaller in size than the second flow path (46), and the liquid in the first flow path (45) being volatilized by the first heating section (40) before the liquid in the second flow path (46) is volatilized by the second heating section (41).

30. A method according to claim 28 or 29 wherein the aerosol generator (21) includes a controller (48), a valve (35) and a sensor (37), the method including:
sensing a delivery condition with the sensor (37);
sending a signal to the controller (48)corresponding to detection of the delivery condition;
opening the valve (35) for delivery of a predetermined volume of liquid to the first (45) and second (46) flow paths when the controller (48) receives the signal;
supplying power to first (40) and second (41) heating sections which heat the liquid in the first (45) and second (46) flow paths; and
closing the valve (35) when the predetermined volume of fluid has been delivered to the first (45) and second (46) flow paths.

31. A method according to claim 28, 29 or 30 wherein a first amount of the volatilized liquid is ejected from a first outlet (25A) and a second amount of volatilized liquid is ejected from a second outlet (25B), the first amount being smaller than the second amount.

## Patentansprüche

1. Aerosolgenerator (21) (121) zum Erzeugen von zerstäubtem Fluid, der Folgendes umfasst:
eine Flüssigkeitsversorgung (33),
einen Strömungsdurchgang mit wenigstens einem ersten (45) und einem zweiten (46) Strömungsweg, einem mit der Flüssigkeitsversorgung (33) in Fluidkommunikation befindlichen Einlass (31) und wenigstens einem Auslass (25A, B),
eine Heizanordnung (23) mit einem ersten (40) und einem zweiten (41) Heizabschnitt, wobei der erste Heizabschnitt (40) dafür ausgelegt ist, Flüssigkeit in dem ersten Strömungsweg (45) ausreichend zu erhitzen, um die Flüssigkeit zum Bilden einer zerstäubten Flüssigkeit zu zerstäuben, die aus dem wenigstens einen Auslass (25A) ausgestoßen wird, und der zweite Heizabschnitt (41) dafür ausgelegt ist, Flüssigkeit in dem zweiten Strömungsweg (46) ausreichend zu erhitzen, um die Flüssigkeit zum Bilden einer zerstäubten Flüssigkeit zu zerstäuben, die aus dem wenigstens einen Auslass (25B) ausgestoßen wird,
**dadurch gekennzeichnet, dass** der erste (45) und der zweite (46) Strömungsweg des Strömungsdurchgangs an zuströmseitigen Enden davon in Fluidverbindung stehen und dass der genannte Einlass ein einzelner Einlass (31) zum Zuführen von Flüssigkeit zu dem ersten und dem zweiten Strömungsweg ist.

2. Aerosolgenerator (21) nach Anspruch 1, bei dem die Heizanordnung (23) wenigstens eine Schicht aus Widerstandsheizungsmaterial aufweist und/oder der wenigstens erste (45) und zweite (46) Strömungsweg kapillare Dimensionen haben.

3. Aerosolgenerator (21) nach Anspruch 2, bei dem der erste (40) und der zweite (41) Heizabschnitt den gleichen oder jeweils einen anderen elektrischen Widerstand haben.

4. Aerosolgenerator (21) nach Anspruch 2 oder 3, bei dem das Widerstandsheizungsmaterial eine Platinbeschichtung umfasst.

5. Aerosolgenerator (21) nach Anspruch 2, 3 oder 4, bei dem die Heizanordnung (23) einen Zwischenabschnitt aus Widerstandsheizungsmaterial beinhaltet, der zwischen dem ersten (40) und dem zweiten (41) Heizabschnitt verläuft.

6. Aerosolgenerator (21) nach Anspruch 2, 3, 4 oder 5, bei dem der erste (40) und der zweite (41) Heizabschnitt von einer einzelnen elektrischen Stromquelle (29) angetrieben wird.

7. Aerosolgenerator (21) nach einem der vorhergehenden Ansprüche, der einen ersten (25A) und einen zweiten (25B) Auslass aufweist.

8. Aerosolgenerator (21) nach einem der vorhergehenden Ansprüche, wobei der erste Heizabschnitt (40) einen anderen Querschnitt als der zweite Heizabschnitt (41) hat.

9. Aerosolgenerator (21) nach Anspruch 8, bei dem der erste Heizabschnitt (40) einen kleineren Querschnitt als der zweite Heizabschnitt (41) hat, so dass der erste Heizabschnitt (40) Flüssigkeit in dem ersten Strömungsweg (45) schneller verdunstet, als der zweite Heizabschnitt (41) Flüssigkeit in dem zweiten Strömungsweg (46) verdunstet, wenn dem ersten und dem zweiten Strömungsweg Flüssigkeit zugeführt wird.

10. Aerosolgenerator (21) nach einem der vorhergehenden Ansprüche, der ferner eine Steuerung (48), ein Ventil (35) und einen Sensor (37) aufweist, wobei der Sensor (37) eine Abgabebedingung erkennt, die der Abgabe eines vorbestimmten Aerosolvolumens entspricht, wobei die Steuerung (48) programmiert ist zum Öffnen des Ventils (35), um Flüssigkeit an den ersten (45) und den zweiten (46) Strömungsweg abzugeben, wenn die Abgabebedingung von dem Sensor (37) erfasst wird, und zum Aktivieren der Heizabschnitte (40, 41) zum Verdunsten von Flüssigkeit in dem ersten (45) und dem zweiten (46) Strömungsweg.

11. Aerosolgenerator (21) nach einem der vorhergehenden Ansprüche, bei dem der erste Heizabschnitt (40) thermisch von dem zweiten Heizabschnitt (41) isoliert ist.

12. Aerosolgenerator (21) nach einem der Ansprüche 1 bis 6, bei dem der erste Heizabschnitt (40) thermisch in den zweiten Heizabschnitt (41) integriert ist.

13. Aerosolgenerator (21) nach einem der vorhergehenden Ansprüche, bei dem der erste (40) und der zweite (41) Heizabschnitt die gleiche oder jeweils eine andere thermische Masse haben.

14. Aerosolgenerator (21) nach einem der vorhergehenden Ansprüche, der ferner ein Mundstück eines Inhalators aufweist, wobei der wenigstens eine Auslass (25A, B) sich in dem Mundstück befindet, um in dem Mundstück ein Aerosol zu bilden, wenn das verdunstete flüssige Material aus dem wenigstens einen Auslass (25A, B) ausgestoßen wird.

15. Aerosolgenerator (21) nach einem der vorhergehenden Ansprüche, bei dem der erste Strömungsweg (45) so bemessen ist, dass er weniger als die Hälfte einer Flüssigkeitsmenge aufnimmt, die in dem zweiten Strömungsweg (46) enthalten ist.

16. Aerosolgenerator (21) nach einem der vorhergehenden Ansprüche, bei dem der erste (40) und der zweite (41) Heizabschnitt während der Abgabe eines festgelegten Flüssigkeitsvolumens an den Strömungsdurchgang verschiedene Volumen zerstäubter Flüssigkeit aus dem wenigstens einen Auslass (25A, B) ausstoßen.

17. Aerosolgenerator (21) nach einem der vorhergehenden Ansprüche, bei dem der erste (45) und der zweite (46) Strömungsweg einen ersten und einen zweiten Kanal zwischen einer Grundplatte (24) und einer Abdeckplatte (26) umfassen.

18. Aerosolgenerator (21) nach Anspruch 17, bei dem der erste Heizabschnitt (40) eine Schicht aus Widerstandsheizungsmaterial in dem ersten Kanal umfasst und der zweite Heizabschnitt (41) eine Schicht aus Widerstandsheizungsmaterial in dem zweiten Kanal umfasst.

19. Aerosolgenerator (21) nach einem der vorhergehenden Ansprüche, bei dem der erste Heizabschnitt (40) unabhängig von dem zweiten Heizabschnitt (41) betrieben wird.

20. Aerosolgenerator (21) nach einem der vorhergehenden Ansprüche, bei dem der erste (40) und der zweite (41) Heizabschnitt ebene Streifen aus Heizungsmaterial mit elektrischem Widerstand umfassen.

21. Aerosolgenerator (21) nach Anspruch 20, wobei die Streifen aus Heizungsmaterial mit elektrischem Widerstand parallel zueinander sind.

22. Aerosolgenerator (21) nach Anspruch 20 oder 21, bei dem der erste (40) und der zweite (41) Heizabschnitt durch einen Zwischenabschnitt (42) miteinander verbunden sind, der einen ebenen Streifen aus Heizungsmaterial mit elektrischem Widerstand umfasst.

23. Aerosolgenerator (21) nach einem der vorhergehenden Ansprüche, bei dem der erste (45) und der zweite (46) Strömungsweg einen ersten und einen zweiten Kanal in einem Keramiksubstrat umfassen.

24. Aerosolgenerator (21) nach Anspruch 23, bei dem die genannten Kanäle in dem Keramiksubstrat eine Tiefe von weniger als oder gleich 10 mm haben.

25. Aerosolgenerator (21) nach Anspruch 23 oder 24, bei dem der erste (40) und der zweite (41) Heizabschnitt Widerstandsheizungsmaterial in den Kanälen umfassen.

26. Aerosolgenerator (21) nach Anspruch 23, 24 oder 25, wobei wenigstens eine elektrische Durchführung (30A, B) durch das Keramiksubstrat verläuft und die Heizabschnitte mit Strom versorgt.

27. Aerosolgenerator (21) nach Anspruch 23, 24, 25 oder 26, der eine erste Durchführung (30A), die mit einem abströmseitigen Ende des ersten Heizabschnitts (40) verbunden ist, und eine zweite Durchführung (30B), die mit einem abströmseitigen Ende des zweiten Heizabschnitts (41) verbunden ist, aufweist.

28. Verfahren zum Erzeugen eines Aerosols, umfassend:
a) Zuführen eines Materials in flüssiger Form zu einem Einlass (31) eines Aerosolgenerators (21), der einen Strömungsdurchgang hat, der einen ersten (45) und einen zweiten (46) Strömungsweg hat, und
b) Erhitzen der Flüssigkeit in dem ersten (45) und zweiten (46) Strömungsweg auf eine Temperatur, die ausreicht, um die Flüssigkeit zu verdunsten und verdunstete Flüssigkeit aus wenigstens einem Auslass (25A, B) auszustoßen,
**dadurch gekennzeichnet, dass** der erste (45) und der zweite (46) Strömungsweg des Strömungsdurchgangs an zuströmseitigen Enden davon in Fluidverbindung stehen und dass der genannte Einlass ein einzelner Einlass (31) zum Zuführen von Flüssigkeit zu dem ersten und dem zweiten Strömungsweg ist.

29. Verfahren nach Anspruch 28, bei dem die Heizung durchgeführt wird, indem ein erster (40) und ein zweiter (41) Heizabschnitt bestromt werden, wobei der erste Strömungsweg (45) eine kleinere Größe als der zweite Strömungsweg (46) hat, und die Flüssigkeit in dem ersten Strömungsweg (45) von dem ersten Heizabschnitt (40) verdunstet wird, bevor die Flüssigkeit in dem zweiten Strömungsweg (46) von dem zweiten Heizabschnitt (41) verdunstet wird.

30. Verfahren nach Anspruch 28 oder 29, bei dem der Aerosolgenerator (21) eine Steuerung (48), ein Ventil (35) und einen Sensor (37) aufweist, wobei das Verfahren Folgendes beinhaltet:
Erfassen einer Abgabebedingung mit dem Sensor (37),
Senden eines der Erkennung der Abgabebedingung entsprechenden Signals an die Steuerung (48),
Öffnen des Ventils (35) zur Abgabe eines vorbestimmten Flüssigkeitsvolumens an den ersten (45) und den zweiten (46) Strömungsweg, wenn die Steuerung (48) das Signal empfängt,
Versorgen des ersten (40) und des zweiten (41) Heizabschnitts, die die Flüssigkeit im ersten (45) und im zweiten (46) Strömungsweg erhitzen, mit Strom und
Schließen des Ventils (35), wenn das vorbestimmte Fluidvolumen an den ersten (45) und den zweiten (46) Strömungsweg abgegeben wurde.

31. Verfahren nach Anspruch 28, 29 oder 30, bei dem eine erste Menge der verdunsteten Flüssigkeit aus einem ersten Auslass (25A) ausgestoßen wird und eine zweite Menge verdunsteter Flüssigkeit aus einem zweiten Auslass (25B) ausgestoßen wird, wobei die erste Menge kleiner als die zweite Menge ist.

## Revendications

1. Générateur d'aérosol (21) pour générer un fluide vaporisé comprenant :
une alimentation de liquide (33) ;
un passage d'écoulement comportant au moins un premier (45) et un second (46) chemin d'écoulement, une admission (31) en communication fluidique avec l'alimentation de liquide (33) et au moins une sortie (25A, B) ;
un agencement de chauffage (23) comportant des première (40) et seconde (41) sections chauffantes, la première section chauffante (40) étant adaptée pour chauffer suffisamment un liquide dans le premier chemin d'écoulement (45) pour le vaporiser de façon à former un liquide vaporisé éjecté de l'au moins une sortie (25A), et la seconde section chauffante (41) étant adaptée pour chauffer suffisamment le liquide dans le second chemin d'écoulement (46) pour le vaporiser de façon à former un liquide vaporisé éjecté de l'au moins une sortie (25B)
**caractérisé en ce que** les premier (45) et second (46) chemins d'écoulement du passage d'écoulement sont en connexion fluidique à des extrémités amont de ceux-ci et **en ce que** ladite admission est une admission unique (31) pour alimenter le liquide dans les premier et second chemins d'écoulement.

2. Générateur d'aérosol (21) selon la revendication 1, dans lequel l'agencement de chauffage (23) comprend au moins une couche de matériau chauffant par résistance et/ou les au moins premier (45) et second (46) chemins d'écoulement ont des dimensions capillaires.

3. Générateur d'aérosol (21) selon la revendication 2, dans lequel les première (40) et seconde (41) sections chauffantes ont des résistances électriques identiques ou différentes.

4. Générateur d'aérosol (21) selon la revendication 2 ou 3, dans lequel le matériau chauffant par résistance comprend un revêtement de platine.

5. Générateur d'aérosol (21) selon la revendication 2, 3 ou 4, dans lequel l'agencement de chauffage (23) comporte une section intermédiaire de matériau chauffant par résistance s'étendant entre les première (40) et seconde (41) sections chauffantes.

6. Générateur d'aérosol (21) selon la revendication 2, 3, 4 ou 5, dans lequel les première (40) et seconde (41) sections chauffantes sont alimentées électriquement par une même source de puissance électrique (29).

7. Générateur d'aérosol (21) selon l'une quelconque des revendications précédentes, comprenant des première (25A) et seconde (25B) sorties.

8. Générateur d'aérosol (21) selon l'une quelconque des revendications précédentes, dans lequel la première section chauffante (40) a une superficie en coupe différente de celle de la seconde section chauffante (41).

9. Générateur d'aérosol (21) selon la revendication 8, dans lequel la première section chauffante (40) a une superficie en coupe inférieure à celle de la seconde section chauffante (41) de telle sorte que la première section chauffante (40) volatilise le liquide dans le premier chemin d'écoulement (45) plus rapidement que la seconde section chauffante (41) volatilise le liquide dans le second chemin d'écoulement (46) quand le liquide est alimenté dans les premier et second chemins d'écoulement.

10. Générateur d'aérosol (21) selon l'une quelconque des revendications précédentes, comprenant en outre une unité de commande (48), une vanne (35) et un capteur (37), le capteur (37) détectant une condition de délivrance correspondant à la délivrance d'un volume prédéterminé d'aérosol, l'unité de commande (48) étant programmée pour ouvrir la vanne (35) de façon à distribuer le liquide aux premier (45) et second (46) chemins d'écoulement quand la condition de délivrance est détectée par le capteur (37) et pour activer les sections chauffantes (40, 41) afin de volatiliser le liquide dans les premier (45) et second (46) chemins d'écoulement.

11. Générateur d'aérosol (21) selon l'une quelconque des revendications précédentes, dans lequel la première section chauffante (40) est isolée thermiquement de la seconde section chauffante (41).

12. Générateur d'aérosol (21) selon l'une quelconque des revendications 1 à 6, dans lequel la première section chauffante (40) est intégrée thermiquement à la seconde section chauffante (41).

13. Générateur d'aérosol (21) selon l'une quelconque des revendications précédentes, dans lequel les première (40) et seconde (41) sections chauffantes ont des masses thermiques identiques ou différentes.

14. Générateur d'aérosol (21) selon l'une quelconque des revendications précédentes, comprenant en outre un embout buccal d'un inhalateur, l'au moins une sortie (25A, B) étant située à l'intérieur de l'embout buccal de façon à former un aérosol dans l'embout buccal quand la substance liquide volatilisée est éjectée de l'au moins une sortie (25A, B).

15. Générateur d'aérosol (21) selon l'une quelconque des revendications précédentes, dans lequel le premier chemin d'écoulement (45) est dimensionné pour renfermer moins qu'une moitié d'une quantité de liquide contenue dans le second chemin d'écoulement (46).

16. Générateur d'aérosol (21) selon l'une quelconque des revendications précédentes, dans lequel les première (40) et seconde (41) sections chauffantes éjectent différents volumes de liquide vaporisé depuis l'au moins une sortie (25A, B) durant la délivrance d'un volume fixe de liquide au passage d'écoulement.

17. Générateur d'aérosol (21) selon l'une quelconque des revendications précédentes, dans lequel les premier (45) et second (46) chemins d'écoulement comprennent des premier et second canaux entre une plaque de base (24) et une plaque de couverture (26).

18. Générateur d'aérosol (21) selon la revendication 17, dans lequel la première section chauffante (40) comprend une couche de matériau chauffant par résistance dans le premier canal et la seconde section chauffante (41) comprend une couche de matériau chauffant par résistance dans le second canal.

19. Générateur d'aérosol (21) selon l'une quelconque des revendications précédentes, dans lequel la première section chauffante (40) est alimentée électriquement indépendamment de la seconde section chauffante (41).

20. Générateur d'aérosol (21) selon l'une quelconque des revendications précédentes, dans lequel les première (40) et seconde (41) sections chauffantes comprennent des bandes planes de matériau chauffant électriquement résistif.

21. Générateur d'aérosol (21) selon la revendication 20, dans lequel les bandes de matériau chauffant électriquement résistif sont parallèles les unes aux autres.

22. Générateur d'aérosol (21) selon la revendication 20 ou 21, dans lequel les première (40) et seconde (41) sections chauffantes sont interconnectées par une section intermédiaire (42) comprenant une bande plane de matériau chauffant électriquement résistif.

23. Générateur d'aérosol (21) selon l'une quelconque des revendications précédentes, dans lequel les premier (45) et second (46) chemins d'écoulement comprennent des premier et second canaux dans un substrat céramique.

24. Générateur d'aérosol (21) selon la revendication 23, dans lequel lesdits canaux dans le substrat céramique sont d'une profondeur inférieure ou égale à 10 mm.

25. Générateur d'aérosol (21) selon la revendication 23 ou 24, dans lequel les première (40) et seconde (41) sections chauffantes comprennent un matériau chauffant par résistance dans les canaux.

26. Générateur d'aérosol (21) selon la revendication 23, 24 ou 25, dans lequel au moins un orifice d'amenée électrique (30A, B) s'étend à travers le substrat céramique et alimente électriquement les sections chauffantes.

27. Générateur d'aérosol (21) selon la revendication 23, 24, 25 ou 26, comprenant un premier orifice d'amenée électrique (30A) connecté à une extrémité aval de la première section chauffante (40) et un second orifice d'amenée électrique (30B) connecté à une extrémité aval de la seconde section chauffante (41).

28. Procédé de génération d'un aérosol, comprenant :
(a) l'alimentation d'une substance sous forme liquide à une admission (31) d'un générateur d'aérosol (21) ayant un passage d'écoulement comportant un premier (45) et un second (46) chemins d'écoulement ; et
(b) le chauffage du liquide dans les premier (45) et second (46) chemins d'écoulement à une température suffisante pour volatiliser le liquide et éjecter le liquide volatilisé depuis au moins une sortie (25A, B).
**caractérisé en ce que** les premier (45) et second (46) chemins d'écoulement du passage d'écoulement sont en connexion fluidique à des extrémités amont de ceux-ci et **en ce que** ladite admission est une admission unique (31) pour alimenter le liquide dans les premier et second chemins d'écoulement.

29. Procédé selon la revendication 28, dans lequel le chauffage est exécuté en excitant des première (40) et seconde (41) sections chauffantes, le premier chemin d'écoulement (45) étant d'une taille inférieure à celle du second chemin d'écoulement (46), et le liquide dans le premier chemin d'écoulement (45) étant volatilisé par la première section chauffante (40) avant que le liquide dans le second chemin d'écoulement (46) soit volatilisé par la seconde section chauffante (41).

30. Procédé selon la revendication 28 ou 29, dans lequel le générateur d'aérosol (21) comporte une unité de commande (48), une vanne (35) et un capteur (37), le procédé comportant :
la détection par le capteur (37) d'une condition de délivrance ;
l'envoi d'un signal à l'unité de commande (48) correspondant à la détection de la condition de délivrance ;
l'ouverture de la vanne (35) pour la délivrance d'un volume prédéterminé de liquide aux premier (45) et second (46) chemins d'écoulement quand l'unité de commande (48) reçoit le signal ;
l'alimentation électrique des première (40) et seconde (41) sections chauffantes qui chauffent le liquide dans les premier (45) et second (46) chemins d'écoulement ; et
la fermeture de la vanne (35) quand le volume de fluide prédéterminé a été délivré aux premier (45) et second (46) chemins d'écoulement.

31. Procédé selon la revendication 28, 29 ou 30, dans lequel une première quantité du liquide volatilisé est éjectée d'une première sortie (25A) et une seconde quantité de liquide volatilisé est éjectée d'une seconde sortie (25B), la première quantité étant inférieure à la seconde quantité.
